# EUROPÄISCHE PATENTSCHRIFT

(11) **EP 2 259 808 B1**
(45) Veröffentlichungstag und Bekanntmachung des Hinweises auf die Patenterteilung: **28.09.2016**
(21) Anmeldenummer: 09715523.8
(22) Anmeldetag: 17.02.2009
(51) Int. Cl.: C08G 18/48, C08G 18/73, C08G 18/76, C08G 18/42, C08G 18/10, A61L 31/14, C08G 18/38, A61L 31/06

(54) **POLYHARNSTOFF-SYSTEME UND DEREN ANWENDUNG ALS POSTOPERATIVE ADHÄSIONSBARRIEREN**
POST-OPERATIVE ADHESION BARRIERS
BARRIÈRES D'ADHÉSION POSTOPÉRATOIRES

(30) Priorität: 28.02.2008 EP 08003620
(43) Veröffentlichungstag der Anmeldung: 15.12.2010
(60) Teilanmeldung: 16170678.3
(73) Patentinhaber: Adhesys Medical GmbH, 52074 Aachen (DE)
(72) Erfinder: HECKROTH, Heike, 51519 Odenthal (DE); NEFZGER, Hartmut, 50259 Pulheim (DE); WAMPRECHT, Christian, 41472 Neuss (DE)
(74) Vertreter: Davepon, Björn
(86) Internationale Anmeldenummer: PCT/EP2009/001085
(87) Internationale Veröffentlichungsnummer: WO 2009/106245

(56) Entgegenhaltungen:
- EP-A1- 2 011 808
- US-A- 5 243 012
- US-A- 6 013 755
- US-A1- 2004 067 315

## Beschreibung

Die vorliegende Erfindung betrifft neuartige Adhäsionsbarrieren auf Basis hydrophiler Polyisocyanat-Prepolymere für den Einsatz in der Chirurgie.

Zu den häufigsten Komplikationen nach Eingriffen im Bauch- und Beckenbereich gehören Adhäsionen. Adhäsionen sind fibröse Bänder, die im Allgemeinen innerhalb der ersten sieben Tage nach einer Operation im Rahmen des Heilungsprozesses entstehen. Dadurch wachsen Gewebe und Organe, die normalerweise voneinander separiert sind, zusammen, wodurch eine Vielzahl von Komplikationen wie z.B. chronische Schmerzen, Unfruchtbarkeit oder ein lebensbedrohlicher Darmverschluss auftreten können. Zur Vermeidung derartiger Komplikationen wurden in den letzten Jahren Produkte entwickelt, die die Bildung von Adhäsionen verringern können. Der Erfolg war bislang jedoch limitiert.

Methoden zur Prävention von Adhäsionen sind das Auswaschen der peritonealen Kavität, die Anwendung pharmakologisch aktiver Agentien wie antiinflammatorisch oder fibrinolytisch wirksamer Verbindungen sowie die Applikation mechanisch wirkender Barrieren zur Trennung des Gewebes. Adhäsionsbarrieren bestehen aus einem inerten oder absorbierbaren Material, das auf die entsprechenden Organe aufgetragen wird. Untersucht wurde eine Vielzahl von Materialien wie Polysaccharide (US 4886787, US 5356883), Hyaluronsäure (US 4141973, US 5246698), Alginate (US 5266326), Chitin (US 5093319), Chitosan (US 4532134, US 5679658), Xanthan (US 4994277), Dextran (US 5605938), Zellulose und deren Derivate (Journal of spinal disorders & techniques (2006), 19(4), 270-5), Humanes Serum Albumin (US 5583114), Collagen (US 2005175659), Glucosamine (US 5462976), Polyoxyalkylencopolymere (US 4911926, US 5366735, US 5135751, US 5681576), Polyester (US 5612052, US 6136333) etc. Ein Großteil dieser Materialien wurde wegen mangelnder Wirksamkeit, fehlender Bioabbaubarkeit oder aufgrund von Interaktionen mit der Wundheilung nicht kommerziell entwickelt.

Kommerziell erhältlich sind Produkte in Membranform wie INTERCEED™ (Johnson & Johnson), SEPRAFILM™ (Genzyme Corp.) und REPEL-CV™ (Life Medical Corp.) die innerhalb von 28 Tagen absorbiert werden. Da die Barrieren auf das entsprechende Organ aufgelegt werden, besteht jedoch die Gefahr des Verrutschens.

Barrieren, die wie die Hyaluronsäurederivate SEPRACOAT™ (GenzymeCorp.) und LUBRICOAT™ (Lifecore Biomedical Inc.) als Flüssigkeit appliziert werden, werden oft zu schnell vom Körper abgebaut, wodurch die Barrierewirkung eingeschränkt ist. Zusätzlich besteht die Gefahr der Migration, so dass die Schutzwirkung entfällt.

Hydrogele sind wasserhaltige Polymere, deren Ketten kovalent zu einem dreidimensionalen Netzwerk verknüpft sind. In Wasser quellen sie schnell und unter starker Volumenzunahme. Aufgrund ihres hohen Wassergehaltes werden sie als Adhäsionsbarrieren untersucht. Neben den auf natürlichen Polysacchariden basierenden Hydrogelen (Alginate, Hyaluronsäure) sind insbesondere hydrophile Polyethylenglykol-basierte Systeme (US 2005266086, DE-A 69929278, US 7025990, US 6514534, US 2003/0077242), wie das kommerziell erhältliche SPRAYGEL™ (Confluent Surgical) intensiv erforscht worden. Nachteilig erwiesen sich hierbei jedoch die mitunter zu hohe Abbaugeschwindigkeit und die Azidität der Abbauprodukte bei Verwendung von Milchsäure- basierten Polyestern. Neben der Polyethylenglykolbasierten Hydrogelbildung findet die Redox-System initiierte radikalische Polymerisation häufige Erwähnung (WO 0009087, US 20030077242, US 20050271727). Als Redoxinitiatoren werden u.a. Ascorbinsäure und Peroxide eingesetzt. Neben möglichen Gewebeirritationen ergibt sich die Problematik der wässrigen Konsistenz der beiden Reaktionspartner, wodurch eine Haftung auf dem entsprechenden Organ nicht gegeben ist.

Isocyanat gekappte Polymere wie Polyester- und Polyetherurethane werden in US 2004/0068078 und WO 2004/021983 unter anderem als postoperative Adhäsionsbarrieren beschrieben. Als Isocyanate werden bevorzugt TDI (Toluylendiisocyanat) und IPDI (Isophorondiisocyanat) eingesetzt, wobei die Prepolymere einen Gehalt an monomeren Polyisocyanaten wie TDI von 0,05 bis 1 mEq enthalten, um eine gute Adhäsion am Gewebe zu bewirken. Bei Anwesenheit biologischer Flüssigkeiten oder bei bestimmten Gewebetypen sollten bevorzugt größere Mengen hiervon vorhanden sein. Die Adhäsion erfolgt unter anderem durch Reaktion des Isocyanates mit dem Gewebe. Monomere Isocyanate sind jedoch bekannt dafür, neben Gewebereizungen zu einer Sensibilisierung und damit zu allergischen Reaktionen zu führen. Die Reaktionsgeschwindigkeit des Prepolymers auf dem Gewebe ist bei Verwendung aliphatischer Isocyanate wie HDI stark verlangsamt, so dass ein solches System für die klinische Anwendung nicht praktikabel ist.

US 7129300 beschreibt die Herstellung absorbierbarer 2-Komponentensysteme bestehend aus einem Polyethylenoxid mit zwei oder mehr Amin-Substituenten und einem bioabbaubaren Diisocyanat beziehungsweise einem Isocyanat gekappten Polyethylenoxid mit einem absorbierbaren Diamin.

WO 2006/010278 beschreibt die Herstellung und Verwendung von Polyurethan-Prepolymeren und Polyurethanacrylaten basierend auf aliphatischen Isocyanaten wie HDI. Als Kettenverlängerer (Härter) werden niedermolekulare Diole, Diamine, Triole. Triamine oder Oligomere sowie physiologisch aktive Verbindungen verwendet. Als Katalysator dienen organische Zink-, Zinn-, Magnesium- und Eisenverbindungen. Eingesetzt werden kann die Erfindung unter anderem als Adhäsionsbarriere aber auch für verschiedene Implantate. Die Verwendung eines Katalysators führt jedoch im Allgemeinen zu einer starken Beschleunigung der Aushärtungsgeschwindigkeit des Polymers, was mit einer erhöhten Wärmeentwicklung einhergeht. Dadurch ist die Anwendung auf inneren Organen eingeschränkt.

EP-A 1719530 beschreibt die Verwendung Isocyanat- gekappter Polyester-Makromere basierend auf aliphatischen Dicarbonsäuren und Dihydroxykomponenten wie Polyalkylenoxiden oder Polyethylenglykolen. Als mögliche Isocyanate werden aromatische, aliphatische und alicyclische Isocyanate beschrieben. Prepolymere aus Aromaten- basierten Isocyanaten wie TDI (wie in den Beispielen aufgeführt) haben eine angegebene Vernetzungszeit auf Gewebe von 1-10 min. Der Einsatz Aromaten- basierter Isocyanate ist für die Anwendung im Körper, bei der wie bei den Adhäsionsbarrieren das Produkt vollständig abgebaut wird, jedoch aufgrund der entstehenden Spaltprodukte als kritisch zu betrachten. Auf aliphatischen Isocyanaten basierte Systeme zeigen allerdings erfahrungsgemäß eine nur ungenügende Reaktivität und damit eine zu langsame Aushärtungszeit, die für die Anwendung *in vivo* nicht praktikabel ist. Zusätzlich sind die Viskositäten der in EP-A 1719530 aufgeführten Verbindungen mit im Durchschnitt 60.000 mPas zu hoch, um applizierbar zu sein, so dass ein Lösungsmittel verwendet werden muss.

In WO 2007/067624 wird ein bioabsorbierbares 2-Komponentensystem bestehend aus einem Isocyanatprepolymer basierend auf Glycolid, Lactid, ε-Caprolacton, p-Dioxanon, Trimethylcarbonat und Polyalkylenoxid (z.B. Polyethylenglycole) beschrieben. Als zweite Komponente wird ein Polyamin eingesetzt. Bei Applikation beider Komponenten auf das Gewebe bildet sich ein Gel, welches als Klebstoff oder Adhäsionsbarriere eingesetzt werden kann. Die Prepolymere sind jedoch extrem hochviskos, so dass ohne Lösungsmittelzusatz eine Applikation nur schwer möglich sein wird. Als mögliche Lösungsmittel werden u.a. Wasser, Alkohole und Ketone angegeben. Hydroxylgruppenhaltige Lösungsmittel bergen jedoch das Problem der schnellen Reaktion mit dem Prepolymer, so dass die Gefahr der Vergelung besteht. Die Verarbeitungszeit kann zudem extrem schnell werden und dadurch die Verarbeitung problematisch machen. Der Einsatz von Lösungsmitteln ist allgemein beim Einsatz *in vivo* in den meisten Fällen aufgrund von möglicher Cytotoxizität sowie Wechselwirkung mit dem Gewebe als kritisch zu erachten.

Eine grundsätzliche Eignung von Asparaginsäureestern zur Vernetzung von Prepolymeren ist im Stand der Technik im Rahmen von Oberflächenbeschichtungen bekannt und in DE-A 10246708 oder EP-A 1081171 beschrieben.

EP 2 011 808 beschreibt bereits Wundkleber auf Basis einer Kombination hydrophiler Polyisocyanat-Prepolymere und Aspartaten als Härter. Die Prepolymere basieren auf Polyetherpolyolen und sind daher nicht innerhalb von 6 bis 12 Monaten bioabbaubar. Zudem sind die beschriebenen Systeme starke Klebstoffe und daher als Adhäsionsbarriere ungeeignet.

Die der vorliegenden Erfindung zugrunde liegende Aufgabe bestand nun darin, eine Adhäsionsbarriere herzustellen, die:
- einen flexiblen Film auf den zu schützenden Organen/Gewebe bildet
- auf den zu schützenden Organen/Gewebe adhäriert
- in einem Zeitfenster von bis zu 6 Monaten biologisch abbaubar ist
- biokompatibel ist
- Abbauprodukte ohne Zell- und Gewebetoxizität bildet
- ein der Anwendung angepasstes rasches Verfestigungsverhalten zeigt
- keine wesentliche, gewebeschädigende Exothermie beim Aushärten zeigt
- durch eine eingestellte Viskosität leicht applizierbar ist und nicht in tiefere Gewebeschichten eindringen kann

Unter Gewebe im Rahmen der vorliegenden Erfindung werden Zellverbände verstanden, die aus Zellen gleicher Gestalt und Leistung bestehen wie Epithelgewebe, Myokard, Bindeoder Stützgewebe, Muskeln, Nerven und Knorpel. Hierzu gehören ebenfalls alle aus Zellverbänden aufgebaute Organe wie Leber, Niere, Lunge, Herz, Uterus etc.

Es wurde nun gefunden, dass diese Aufgabe durch eine Kombination von isocyanatfunktionellen Polyester-Prepolymeren auf Basis aliphatischer Isocyanate mit Restmonomergehalten von weniger als 1 Gew.-% in Kombination mit aminofunktionellen Asparaginsäureestern gelöst werden konnte. Für die Vernetzung auf dem Gewebe kann dabei auf Katalysatoren für die Vernetzung verzichtet werden.

Gegenstand der vorliegenden Erfindung ist daher die Verwendung eines Polyharnstoff-Systems zur Herstellung eines Mittels zur Erzeugung einer postoperativen Adhäsionsbarriere, wobei das Polyharnstoff-System umfasst
A) aminofunktionelle Asparaginsäureester der allgemeinen Formel (I) 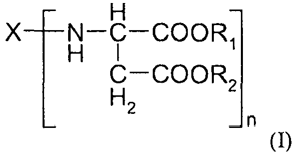 wobei
   - X: ein n-wertiger organischer Rest ist, der durch Entfernung der primären Aminogruppen eines n-wertigen Amins erhalten wird,
   - R₁, R₂: gleiche oder verschiedene organische Reste sind, die keinen Zerewitinoffaktiven Wasserstoff aufweisen und
   - n: eine ganze Zahl von mindestens 2 ist.
   und
B) isocyanatfunktionelle Prepolymere mit Restmonomergehalten von weniger als 1 Gew.-%, bevorzugt von weniger als 0,1 Gew.-% und besonders bevorzugt von weniger als 0,03 Gew.-% erhältlich durch Umsetzung von
B1) aliphatischen Isocyanaten mit
B2) einer Polyolkomponente mit zahlenmittleren Molekulargewichten von ≥ 400 g/mol und mittleren OH-Funktionalitäten von 2 bis 6, die Polyesterpolyole und/oder Polyester-Polyether-Polyole sowie gegebenenfalls Polyetherpolyole enthält und/oder
C) gegebenenfalls organische Füllstoffe, die eine nach DIN 53019 gemessenen Viskosität bei 23°C im Bereich von 10 bis 6000 mPas aufweisen und
D) gegebenenfalls Umsetzungsprodukte von isocyanatfunktionellen Prepolymeren gemäß der Definition der Komponente B) mit Asparaginsäureestern gemäß Komponente A) und/oder organischen Füllstoffen gemäß C).

Zur Definition von Zerewitinoff-aktivem Wasserstoff wird auf Römpp Chemie Lexikon, Georg Thieme Verlag Stuttgart verwiesen. Bevorzugt werden unter Gruppen mit Zerewitinoff-aktivem Wasserstoff OH, NH oder SH verstanden.

Bevorzugt sind in Formel (I):
R₁, R₂ gleiche oder verschiedene gegebenenfalls verzweigte oder cyclische organische Reste, die keinen Zerewitinoff-aktiven Wasserstoff aufweisen, mit 1 bis 20, bevorzugt 1 bis 10 Kohlenstoffatomen, besonders bevorzugt Methyl- oder Ethylgruppen, ,
n eine ganze Zahl von 2 bis 4 und
X ein n-wertiger organischer gegebenenfalls verzweigter oder cyclischer organischer Rest mit 2 bis 20, bevorzugt 5 bis 10 Kohlenstoffatomen, der durch Entfernung der primären Aminogruppen eines n-wertigen primären Amins erhalten wird.

Selbstverständlich können auch Gemische von mehreren Asparaginsäureestern verwendet werden, so dass n in Formel (I) auch einen nicht ganzzahligen Mittelwert darstellen kann.

Die Herstellung der aminofunktionellen Polyasparaginsäureester A) erfolgt in bekannter Weise durch Umsetzung der entsprechenden primären mindestens difunktionellen Amine X(NH₂)ₙ mit Malein- oder Fumarsäureestern der allgemeinen Formel

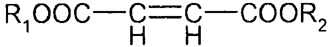

Bevorzugte Malein- oder Fumarsäureester sind Maleinsäuredimethylester, Maleinsäurediethylester, Maleinsäuredibutylester und die entsprechenden Fumarsäureester.

Bevorzugte primäre mindestens difunktionelle Amine X(NH₂)ₙ sind Ethylendiamin, 1,2-Diaminopropan, 1,4-Diaminobutan, 1,3-Diaminopentan, 1,5-Diaminopentan, 2-Methyl-1,5-diaminopentan, 1,6-Diaminohexan, 2,5-Diamino-2,5-dimethylhexan, 2,2,4- und/oder 2,4,4-Trimethyl-1,6-diaminohexan, 1,11-Diaminoundecan, 1,12-Diaminododecan, 1-Amino-3,3,5-trimethyl-5-aminomethyl-cyclohexan, 2,4-und/oder 2,6-Hexahydrotoluylendiamin, 2,4'-und/oder 4,4'-Diamino-dicyclohexylmethan, 3,3'-Dimethyl-4,4'-diamino-dicyclohexylmethan, 2,4,4'-Triamino-5-methyl-dicyclohexylmethan und Polyetheramine mit aliphatisch gebundenen primären Aminogruppen mit einem zahlenmittleren Molekulargewicht Mₙ von 148 bis 6000 g/mol.

Besonders bevorzugte primäre mindestens difunktionelle Amine sind 1,3-Diaminopentan, 1,5-Diaminopentan, 2-Methyl-1,5-diaminopentan, 1,6-Diaminohexan, 1,13-Diamino-4,7,10-trioxatridecan. Ganz besonders bevorzugt ist 2-Methyl-1,5-diaminopentan.

In einer bevorzugten Ausführungsform der Erfindung sind R₁ = R₂ = Ethyl, wobei X auf 2-Methyl-1,5-diaminopentan als n-wertigem Amin basiert.

Die Herstellung der aminofunktionellen Asparaginsäureester A) aus den genannten Ausgangsmaterialien erfolgt nach DE-A 69 311 633 bevorzugt innerhalb des Temperaturbereichs von 0 bis 100 °C, wobei die Ausgangsmaterialien in solchen Mengenverhältnissen eingesetzt werden, dass auf jede primäre Aminogruppe mindestens eine, vorzugsweise genau eine olefinische Doppelbindung entfällt, wobei im Anschluss an die Umsetzung gegebenenfalls im Überschuss eingesetzte Ausgangsmaterialien destillativ abgetrennt werden können. Die Umsetzung kann in Substanz oder in Gegenwart geeigneter Lösungsmittel wie Methanol, Ethanol, Propanol oder Dioxan oder Gemischen derartiger Lösungsmittel erfolgen.

Die Systeme werden durch Mischung der Prepolymere B) mit den aminofunktionellen Asparaginsäureestern A) sowie gegebenenfalls den Komponenten C) und/oder D) erhalten. Das Verhältnis von freien oder blockierten Aminogruppen zu freien NCO-Gruppen beträgt bevorzugt 1:1,5, besonders bevorzugt 1:1.

Die Systeme besitzen unmittelbar nach Vermischung der Einzelkomponenten miteinander eine nach DIN 53019 gemessene Scherviskosität bei 23°C von bevorzugt 500 bis 20000 mPas, besonders bevorzugt 500 bis 8000 mPas.

Der Zeitbedarf zur vollständigen Vernetzung und Aushärtung der Adhäsionsbarriere beträgt bei 23°C typischerweise 30 s bis 10 min, bevorzugt 30 s bis 8 min, besonders bevorzugt 1 min bis 5 min.

Die in B) eingesetzten isocyanatfunktionellen Prepolymere sind durch Umsetzung von Isocyanaten B1) mit Polyolen B2) gegebenenfalls unter Zusatz von Katalysatoren sowie Hilfs- und Zusatzstoffen erhältlich.

Die in B1) eingesetzten Isocyanate oder Isocyanatmischungen haben bevorzugt eine mittlere NCO-Funktionalität von 2 bis 2,6, besonders bevorzugt 2 bis 2,4.

In B1) können als Isocyanate beispielsweise monomere aliphatische oder cycloaliphatische Di- oder Triisocyanate wie 1,4-Butylendiisocyanat (BDI), 1,6-Hexamethylendiisocyanat (HDI), Isophorondiisocyanat (IPDI), 2,2,4- und/oder 2,4,4-Trimethylhexamethylendiisocyanat, die isomeren Bis-(4,4'-isocyanatocyclohexyl)-methane oder deren Mischungen beliebigen Isomerengehalts, 1,4-Cyclohexylendiisocyanat, 4-Isocyanatomethyl-1,8-octandiisocyanat (Nonan-triisocyanat), sowie Alkyl-2,6-diisocyanatohexanoat (Lysindiisocyanat) mit C1-C8-Alkylgruppen eingesetzt werden.

Neben den vorstehend genannten monomeren Isocyanaten können auch deren höhermolekulare Folgeprodukte mit Uretdion-, Isocyanurat-, Urethan-, Allophanat-, Biuret-, Iminooxadiazindion- oder Oxadiazintrionstruktur sowie deren Mischungen eingesetzt werden.

Bevorzugt werden in B1) Isocyanate der vorstehend genannten Art mit ausschließlich aliphatisch oder cycloaliphatisch gebundenen Isocyanatgruppen oder deren Mischungen eingesetzt.

Ganz besonders bevorzugt wird in B1) Hexamethylendiisocyanat eingesetzt.

Die in der Polyolkomponente B2) eingesetzten Polyole haben eine mittlere OH-Funktionalität von 2,3 bis 4.

Bevorzugt werden in B2) Polyetheresterpolyole und bevorzugt deren Mischungen mit Polyetherpolyolen eingesetzt.

Solche bevorzugt einzusetzenden Polyetheresterpolyole besitzen ferner eine Hydroxylzahl von 30 bis 140 mg KOH/g, bevorzugt 35 bis 130 mg KOH/g, sowie eine Säurezahl von 0,05 bis 10 mg KOH/g, bevorzugt 0,1 bis 3 mg KOH/g und besonders bevorzugt 0,15 bis 2,5 mg KOH/g.

Erfindungswesentliche Polyetherester sind darüber hinaus bei Raumtemperatur flüssig und weisen eine nach DIN 53019 gemessene Scherviskosität bei 23°C von 200 bis 8000 mPas, bevorzugt 400 bis 4000 mPas auf.

Solche Polyetheresterpolyole werden entsprechend dem Stand der Technik vorzugsweise durch Polykondensation aus Polycarbonsäuren, Anhydriden von Polycarbonsäuren, sowie Estern von Polycarbonsäuren mit leichtflüchtigen Alkoholen, bevorzugt C1 bis C6 Monoolen, wie Methanol, Ethanol, Propanol oder Butanol, mit molar überschüssigem, niedermolekularem und/oder höhermolekularem Polyol hergestellt; wobei als Polyol ethergruppenhaltige Polyole gegebenenfalls in Mischungen mit anderen ethergruppenfreien Polyolen eingesetzt werden.

Selbstverständlich können zur Polyetherestersynthese auch Gemische der höhermolekularen und der niedermolekularen Polyole verwendet werden.

Solche molar überschüssigen niedermolekularen Polyole sind Polyole mit Molmassen von 62 bis 299 Dalton, mit 2 bis 12 C-Atomen und Hydroxylfunktionalitäten von mindestens 2, die weiterhin verzweigt oder unverzweigt sein können und deren Hydroxylgruppen primär oder sekundär sind. Diese niedermolekularen Polyole können auch Ethergruppen aufweisen. Typische Vertreter sind Ethylenglykol, Propandiol-1,2, Propandiol-1,3, Butandiol-1,4, Butandiol-2,3, 2-Methyl-propandiol-1,3, Pentandiol-1,5, Hexandiol-1,6, 3-Methyl-pentandiol-1,5, 1,8-Octandiol, 1,10-Decandiol, 1,12-Dodecandiol, Cyclohexandiol, Diethylenglykol, Triethylenglykol und höhere Homologe, Dipropylenglykol, Tripropylenglykol und höhere Homologe, Glycerin, 1,1,1-Trimethylolpropan, sowie Oligo-tetrahydrofurane mit Hydroxylendgruppen. Selbstverständlich können innerhalb dieser Gruppe auch Gemische verwendet werden.

Molar überschüssige höhermolekulare Polyole sind Polyole mit Molmassen von 300 bis 3000 Dalton, die durch ringöffenende Polymerisation von Epoxiden, bevorzugt Ethylen- und/oder Propylenoxid, sowie durch säurekatalysierte, ringöffnende Polymerisation von Tetrahydrofuran, erhalten werden. Zur ringöffnenden Polymerisation von Epoxiden können entweder Alkalihdroxide oder Doppelmetallcyanidkatalysatoren verwendet werden.

Als Starter für ringöffnende Epoxidpolymerisationen können alle mindestens bifunktionellen Moleküle aus der Gruppe der Amine und der o.g. niedermolekularen Polyole verwendet werden. Typische Vertreter sind 1,1,1-Trimethylolpropan, Glycerin, o-TDA. Ethylendiamin, Propylenglykol-1,2, etc. sowie Wasser, einschließlich deren Gemische. Selbstverständlich können innerhalb der Gruppe der überschüssigen höhermolekularen Polyole auch Gemische verwendet werden.

Der Aufbau der höhermolekularen Polyole, soweit es sich um Hydroxylgruppen terminierte Polyalkylenoxide aus Ethylen- und/oder Propylenoxid handelt, kann statistisch oder blockweise erfolgen, wobei auch Mischblöcke enthalten sein können.

Polycarbonsäuren sind sowohl aliphatische als auch aromatische Carbonsäuren, die sowohl cyclisch, linear, verzweigt oder unverzweigt sein können und die zwischen 4 und 24 C-Atome aufweisen

Beispiele sind Bernsteinsäure, Glutarsäure, Adipinsäure, Azelainsäure, Sebacinsäure, 1,10-Decandicarbonsäure, 1,12-Dodecandicarbonsäure, Phthalsäure, Terephthalsäure, Isophthalsäure, Trimellitsäure, Pyromellitsäure. Bevorzugt sind Bernsteinsäure, Glutarsäure, Adipinsäure, Sebacinsäure, Milchsäure, Phthalsäure, Terephthalsäure, Isophthalsäure, Trimellitsäure, Pyromellitsäure. Besonders bevorzugt sind Bernsteinsäure, Glutarsäure und Adipinsäure.

Weiterhin umfasst die Gruppe der Polycarbonsäuren auch Hydroxycarbonsäuren, bzw. deren innere Anhydride, wie z.B. Caprolacton, Milchsäure, Hydroxybuttersäure, Ricinolsäure, usw. Mit umfasst sind weiterhin auch Monocarbonsäuren, insbesondere solche, die über mehr als 10 C-Atome verfügen, wie Sojaölfettsäure, Palmölfettsäure und Erdnussölfettsäure, wobei deren Anteil am gesamten, das Polyetheresterpolyol aufbauenden Reaktionsmischung 10 Gew.-% nicht übersteigt und zusätzlich die dadurch einhergehende Minderfunktionalität durch Mitverwendung von mindestens trifunktionellen Polyolen, sei es auf Seiten der niedermolekularen oder der hochmolekularen Polyole ausgeglichen wird.

Die Herstellung der Polyetheresterpolyol erfolgt entsprechend dem Stand der Technik bei erhöhter Temperatur im Bereich von 120 bis 250°C, zunächst unter Normaldruck, später unter Anlegen von Vakuum von 1 bis 100 mbar, vorzugsweise, aber nicht notwendigerweise unter Verwendung eines Veresterungs- oder Umesterungskatalysators, wobei die Reaktion so weit vervollständigt wird, dass die Säurezahl auf Werte von 0,05 bis 10 mg KOH/g, bevorzugt 0,1 bis 3 mg KOH/g und besonders bevorzugt 0,15 bis 2,5 mg KOH/g absinkt. Weiterhin kann im Rahmen der Normaldruckphase vor dem Anlegen von Vakuum ein Inertgas verwendet werden. Selbstverständlich können alternativ oder für einzelne Phasen der Veresterung auch flüssige oder gasförmige Schleppmittel zum Einsatz kommen. Beispielsweise kann das Reaktionswasser unter Verwendung von Stickstoff als Trägergas, ebenso ausgetragen werden, wie unter Einsatz eines Azeotropschleppmittels, wie z.B. Benzol, Toluol, Xylol, Dioxan, etc.

Die in B2 ggf. als Abmischkomponente eingesetzten Polyetherpolyole haben ein Molekulargewicht von 100 bis 2000 g/mol, bevorzugt 100 bis 1000 g/mol, besonders bevorzugt 100 bis 400 g/mol und bestehen teilweise oder ganz aus Polyethylenoxid Polyolen.

Werden in B2 Polyetherpolyole neben den Polyestern bzw. Polyetherestern eingesetzt, so beträgt ihr Anteil höchstens 70 Gew.-%, bevorzugt höchstens 50 Gew.-% bezogen auf die gesamte Komponente B2.

Bevorzugt beträgt der auf Ethylenoxid zurückführbare Massenanteil der gesamten Komponente B2 bevorzugt 40 bis 95 Gew.-%, besonders bevorzugt 60 bis 90 Gew.-%.

Bevorzugt weist die Komponente B2 ferner eine Estergruppenkonzentration (in Mol pro kg) von 0,5 bis 5,5, besonders bevorzugt 1 bis 3,5 auf.

Die aus B1 und B2 hergestellten Prepolymere haben einen nach DIN EN ISO 11909 gemessenen mittleren NCO-Gehalt von 2 bis 10 Gew.-%, bevorzugt 2,5 bis 8 Gew.-%.

Die in C) eingesetzten organischen flüssigen Füllstoffe sind bevorzugt gemäß Zytotoxizitätsmessung nach ISO 10993 nicht zytotoxisch.

Beispielsweise können als organische Füllstoffe bei 23°C flüssige Polyethylenglykole wie PEG 200 bis PEG 600, deren Mono- bzw. Dialkylether wie PEG 500 Dimethylether, flüssige Polyether- und Polyesterpolyole, flüssige Polyester wie z.B. Ultramoll (Lanxess AG, Leverkusen, DE) sowie Glycerin und seine flüssigen Derivate wie z.B. Triacetin (Lanxess AG, Leverkusen, DE) eingesetzt werden.

Bevorzugt handelt es sich bei den organischen Füllstoffen der Komponente C) um hydroxy- oder aminofunktionelle, bevorzugt rein hydroxyfunktionelle Verbindungen. Besonders bevorzugt sind Polyole. Bevorzugte Polyole sind Polyether und/oder Polyesterpolyole, besonders bevorzugt Polyetherpolyole.

Die bevorzugten organischen Füllstoffe besitzen bevorzugt mittlere OH-Funktionalitäten von 1,5 bis 3, besonders bevorzugt 1,8 bis 2,2, ganz besonders bevorzugt 2,0.

Die bevorzugten organischen Füllstoffe der Komponente besitzen bevorzugt sich wiederholende von Ethylenoxid abgeleitete Einheiten.

Die Viskosität der organischen Füllstoffe beträgt bevorzugt 50 bis 4000 mPas, besonders bevorzugt 50 bis 2000 mPas bei 23°C gemessen nach DIN 53019.

In einer bevorzugten Ausführungsform der Erfindung werden als organische Füllstoffe Polyethylenglykole eingesetzt. Diese haben bevorzugt ein zahlenmittleres Molekulargewicht von 100 bis 1000 g/mol, besonders bevorzugt 200 bis 400 g/mol.

Das Gewichtsverhältnis der Füllstoffkomponente C) zur Aspartatkomponente A) beträgt 0 : 1 bis 20 : 1 , bevorzugt 0 : 1 bis 12 : 1.

Das Gewichtsverhältnis des Füllstoffes bezogen auf die Gesamtmenge der Mischung aus A und B liegt im Bereich von 0 bis 100 %, bevorzugt 0 bis 60 %.

Um das mittlere Equivalentgewicht der insgesamt zur Prepolymervernetzung eingesetzten Verbindungen bezogen auf die NCO-reaktiven Gruppen weiter zu reduzieren, ist es möglich zusätzlich zu den in A) eingesetzten Verbindungen auch die amino- oder hydroxyfunktionellen Umsetzungsprodukte isocyanatfunktioneller Prepolymere mit Asparaginsäureestern und/oder organischen Füllstoffen, sofern diese amino- oder hydroxyfunktionell sind, in einer separaten Vorreaktion herzustellen und dann als höhermolekulare Härterkomponente einzusetzen.

Bevorzugt werden bei der Vorverlängerung Verhältnisse von isocyanatreaktiven Gruppen zu Isocyanatgruppen von 50 zu 1 bis 1,5 zu 1 eingesetzt, besonders bevorzugt 15 zu 1 bis 4 zu 1.

Das dafür einzusetzende isocyanatfunktionelle Prepolymer kann dabei demjenigen der Komponente B) entsprechen oder aber auch abweichend aus den Komponenten, wie sie als mögliche Bestandteile der isocyanatfunktionellen Prepolymere im Rahmen dieser Anmeldung gelistet sind, aufgebaut sein.

Vorteil dieser Modifizierung durch Vorverlängerung ist, dass das Equivalentgewicht und Equivalentvolumen der Härterkomponente in deutlichen Grenzen modifizierbar ist. Dadurch können zur Applikation kommerziell verfügbare 2-Kammerdosiersysteme eingesetzt werden, um ein Klebesystem zu erhalten, das bei bestehenden Verhältnissen der Kammervolumina in das gewünschte Verhältnis von NCO-reaktiven Gruppen zu NCO-Gruppen eingestellt werden kann.

Beschrieben ist ein Verfahren zur Herstellung der Harnstoffsysteme zur Verwendung als Beschichtungsmittel zum Abdecken von Zellgeweben.

Unter Abdecken von Zellgeweben wird die Herstellung postoperativer Adhäsionsbarrieren verstanden.

Im Falle von Beschichtungsmitteln zur Herstellung postoperativer Adhäsionsbarrieren kann es sinnvoll sein, eine oder mehrere der eingesetzten Komponenten A) bis D) einzufärben, um die Barriere leichter sichtbar zu machen.

Bei der *in vivo*-Applikation einer Beschichtung zur Erzeugung einer postoperativen Adhäsionsbarriere werden die notwendigen Komponenten mit Hilfe eines Zweikammerdosiersystems mit einem geeigneten Applikator auf das zu schützende Organ aufgetragen und bilden dort innerhalb von 10 Minuten einen schützenden Polymerfilm. Dieser adhäriert auf der Organoberfläche ohne in das Gewebe einzudringen. Der Film kann mechanisch ohne Schädigung des Gewebes entfernt werden.

### Beispiele:

Sofern nicht abweichend vermerkt beziehen sich alle Prozentangaben auf Gewichtsprozent.

### Beispiel A: (Aspartat-Synthese)

Zu 2 Mol Diethylmaleat wurde unter Stickstoffatmosphäre langsam 1 Mol 2-Methyl-1,5-diaminopentan getropft, so dass die Reaktionstemperatur 60°C nicht überschritt. Anschließend wurde so lange auf 60°C erwärmt, bis kein Diethylmaleat mehr im Reaktionsgemisch nachweisbar war.

### Beispiel B: (Polyestersynthese)

### Rohstoffe

Polyether 1 ist ein auf Propylenglykol-1,2 gestartetes, KOH katalysiertes Polyetherglykol der Fa. BMS AG mit einer Hydroxylzahl von 56 mg KOH/g und mit ca. je 50 Gew.% Ethylen- und Propylenoxideinheiten, wobei die Kettenenden mit Ethylenoxid getippt sind.

Polyether 2 ist ein auf Propylenglykol-1,2 gestartetes, KOH katalysiertes, Hydroxylgruppen terminiertes Polyethylenoxid der Fa. BMS AG mit einer Hydroxylzahl von 190 mg KOH/g.

Polyether 3 ist ein auf 1,1,1-Trimethylolpropan gestartetes, KOH katalysiertes, Hydroxylgruppen terminiertes Polyethylenoxid der Fa. BMS AG mit einer Hydroxylzahl von 550 mg KOH/g.

Die in der Tabelle 1 aufgeführten Angaben zur Estergruppenkonzentration entspricht der Molzahl an Carboxylgruppen, die für 1 kg Produkt eingesetzt wurden.

Die Angabe "Anteil Ethylenoxidgruppen" berechnet sich aus dem Ethylenoxidgruppenanteil der Edukte Polyether 1 (50 Gew.-%), Polyether 2 (100%), Polyether 3 (100%) und Diethylenglykol (100%) bzw. deren Anteil an den Edukten der Polyesterrezeptur.

In einem 6 1 Kolben mit Thermometer, Kolonne, Rücklaufteiler mit Kopfthermometer, absteigendem Kühler und 1-1-Vorlage wurden 98 g (1,07 Mol) Glycerin, 935 g (0,47 Mol) Polyether 1, 1615 g (2,73 Mol) Polyether 2 und 467 g (3,2 Mol) Adipinsäure langsam unter Rühren bei Normaldruck und unter Stickstoff auf 200 °C erhitzt, wobei Reaktionswasser abdestillierte. Nach 5 Std. wurden 68 mg Zinndichlorid-dihydrat zugegeben und dabei langsam Vakuum auf zuletzt 15 mbar angelegt. Nach weiteren 20 Stunden war die Reaktion beendet. Die Analyse ergab die in Tabelle 1 aufgeführten Werte.

**Tabelle 1: Synthese und Charakterisierung erfindungsgemäßer Polyesterpolyole, Ansatzgröße je 3 kg Produkt**

| **Beispiel** | | **B-1** | **B**-**2** | **B**-**3** | **B**-**4** | **B**-**5** | **B**-**6** | **B**-**7** | **B**-**8** | **B-9** | **B**-**10** |
|---|---|---|---|---|---|---|---|---|---|---|---|
| Adipinsäure | [g] | 467 | 426 | 218 | 289 | 562 | | 733 | 493 | 201 | 292 |
| Bernsteinsäure | [g] | | | | | | 473 | | | | |
| Glycerin | [g] | 98 | | 118 | 98 | | | | | 99 | 82 |
| TMP | [g] | | | | | | | | 256 | | |
| Polyether 1 | [g] | 935 | 931 | 1527 | 2150 | | | 954 | 937 | 2748 | 2148 |
| Polyether 2 | [g] | 1615 | 906 | 1191 | 534 | 1735 | 1829 | 326 | 1179 | | 546 |
| Polyether 3 | [g] | | 842 | | | 842 | 842 | 842 | 256 | | |
| Diethylenglykol | [g] | | | | | | | 326 | | | |
| Zinndichlorid-dihydrat | [mg] | 68 | 68 | 68 | 68 | 68 | 68 | 68 | 68 | 68 | 68 |
| Funktionalität | | 3 | 3,5 | 2,5 | 3 | 3,5 | 3,5 | 3,5 | 3,5 | 3 | 3 |
| OH-Zahl, exp. | [mg KOH/g] | 52,1 | 121,4 | 98,3 | 43,5 | 122 | 121 | 120 | 115 | 44,4 | 38,4 |
| Säurezahl | [mg KOH/g] | 0,9 | 0,3 | 0,3 | 1,2 | 0,2 | 0,3 | 0,2 | 0,3 | 0,6 | 0,7 |
| Viskosität | [mPas, 75°C] | 620 | 180 | 100 | 440 | 2110 | 2490 | 3230 | 1850 | 2340 | 4450 |
| Anteil EthylenoxidEinheiten | [Gew.-%] | 67 | 71 | 64 | 52 | 82 | 85 | 69 | 61 | 45 | 53 |
| Estergruppenkonzentration | [Mol/kg] | 2,13 | 1,95 | 1,00 | 1,32 | 2,56 | 2,67 | 3,35 | 2,25 | 0,92 | 1,33 |

### Beispiel C : Prepolymer-Synthese

### Beispiel C-2a ,

236,95 g HDI und 0,4 g Benzoylchlorid wurden in einem 500 ml Vierhalskolben vorgelegt. Innerhalb von 2h wurden bei 80°C 162,6 g des bei 100°C entwässerten Polyesters 2 aus Bsp. B-2 hinzugefügt und 1h nachgerührt. Anschließend wurde durch Dünnschichtdestillation bei 140°C und 0,1 Torr das überschüssige HDI (Hexamethylendiisocyanat) abdestilliert. Man erhielt 280 g des Präpolymers mit einem NCO-Gehalt von 5,67%. Der Restmonomerengehalt betrug < 0,03 % freies HDI.

### Beispiel C-2b, (Prepolymer-Synthese)

281,88 g HDI und 0,4 g Benzoylchlorid wurden in einem 500 ml Vierhalskolben vorgelegt. Innerhalb von 2h wurden bei 80°C eine bei 100°C entwässerte Mischung aus 96,75 g des Polyesters 2 aus Bsp. B-2 und 20,97 g Polyethylenglykol der Molmasse 200 Da (PEG 200) hinzugefügt und 1h nachgerührt. Anschließend wurde durch Dünnschichtdestillation bei 140°C und 0,1 Torr das überschüssige HDI abdestilliert. Man erhielt 311 g des Präpolymers mit einem NCO-Gehalt von 7,88 %. Der Restmonomerengehalt betrug < 0,03 % freies HDI.

### Beispiel C-2c, (Prepolymer-Synthese)

267,82 g HDI und 0,4 g Benzoylchlorid wurden in einem 500 ml Vierhalskolben vorgelegt. Innerhalb von 2h wurden bei 80°C eine bei 100°C entwässerte Mischung aus 91,92 g des Polyesters 2 aus Bsp. B-2 und 39,85 g PEG 400 hinzugefügt und 1 h nachgerührt. Anschließend wurde durch Dünnschichtdestillation bei 140°C und 0,1 Torr das überschüssige HDI abdestilliert. Man erhielt 302 g des Präpolymers mit einem NCO-Gehalt von 7,57 %. Der Restmonomerengehalt betrug < 0,03 % freies HDI.

### Beispiel D: Herstellung der Adhäsionsbarriere

### Beispiel D

10 g des Prepolymers B wurden mit äquivalenten Mengen des aminofunktionellen Asparaginsäureesters (Aspartat A) in einem Becher gut verrührt. Die Reaktionsmischung wurde unmittelbar danach auf Niere, Leber und Muskelgewebe dünn aufgetragen. Aushärtungszeit und -temperatur sowie Haftung auf dem Gewebe wurden bestimmt.

| Beispiel | **D-1** | **D-2a** | **D-2b** | **D-2c** | **D-3** | **D-4** |
|---|---|---|---|---|---|---|
| Prepolymer des Polyesters | **C-1** | **C-2a** | **C-2b** | **C-2c** | **C-3** | **C-4** |
| NCO-Gehalt des Prepolymers [%] gemessen nach DIN EN iso 11909 | 3,06 | 5,67 | 7,88 | 7,57 | 4,38 | 2,57 |
| Aushärtungszeit [min] | 2 | 1 | 2 | 1,5 | 3 | 3 |
| Aushärtungstemperatur [°C] | 35 | 40 | 41 | 40 | 42 | 35 |

| Beispiel | **D-5** | **D-6** | **D-7** | **D-8** | **D-9** | **D-10** |
|---|---|---|---|---|---|---|
| Prepolymer des Polyesters | **C-5** | **C-6** | **C-7** | **C-8** | **C-9** | **C-10** |
| NCO-Gehalt des Prepolymers [%] | 5,95 | 5,92 | 6,0 | 5,72 | 2,01 | 2,07 |
| Aushärtungszeit [min] | 1,5 | 1 | 1 | 1,5 | 2 | 3 |
| Aushärtungstemperatur [°C] | 41 | 43 | 42 | 47 | 28 | 37 |

Bei allen aufgeführten Beispielen hat sich nach den angegebenen Zeiten ein glänzender, transparenter Film gebildet. In allen Fällen wurde eine gute Haftung ohne Eindringen des Polymers in das Gewebe beobachtet. Die Barrieren konnten ohne Verletzung des Gewebes mechanisch entfernt werden.

### Vergleichsbeispiele:

### Beispiel 1

Bei Auftrag des Prepolymers A-1 ohne Aspartatzusatz auf Gewebe fand innerhalb von 30 min keine Aushärtung statt.

### Beispiel 2

Prepolymer A-1 wurde wie in Beispiel B beschrieben mit TDI anstelle von HDI hergestellt. Das erhaltene Prepolymer wurde mit unterschiedlichen Mengen Wasser versetzt und auf Gewebe aufgetragen. Es hat innerhalb von 15 min keine Aushärtung stattgefunden.

### Beispiel 3, Synthese des Polyetherpolyol-Prepolymer aus EP 2 011 808

465 g HDI und 2.35 g Benzoylchlorid wurden in einem 1 1 Vierhalskolben vorgelegt. Innerhalb von 2h wurden bei 80°C 931.8 g eines Polyethers mit einem Ethylenoxidgehalt von 63% und einem Propylenoxidgehalt von 37% (jeweils bezogen auf dem gesamten Alkylenoxidgehalt) gestartet auf TMP (3-funktionell) hinzugefügt und rührte 1h nach. Anschließend wurde durch Dünnschichtdestillation bei 130°C und 0,1 Torr das überschüssige HDI abdestilliert. Man erhielt 980 g (71 %) des Präpolymers mit einem NCO-Gehalt von 2,53%. Der Restmonomerengehalt betrug < 0,03 % HDI.

### Beispiel 4, Adhäsionsbarriere

10 g des Prepolymers aus Beispiel 3 wurden mit äquivalenten Mengen des aminofunktionellen Asparaginsäureesters (Aspartat A) in einem Becher gut verrührt. Die Reaktionsmischung wurde unmittelbar danach auf Niere, Leber und Muskelgewebe dünn aufgetragen. Innerhalb von 2 min hat eine Aushärtung unter Bildung eines transparenten Films stattgefunden. Das Polymer ist dabei in das Gewebe eingedrungen und konnte nicht ohne Beschädigung entfernt werden.

## Patentansprüche

1. Verwendung eines Polyharnstoff-Systems zur Herstellung eines Mittels zur Erzeugung einer postoperativen Adhäsionsbarriere, wobei das Polyharnstoffsystem umfasst
A) aminofunktionelle Asparaginsäureester der allgemeinen Formel (I) 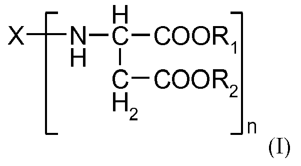 wobei
X ein n-wertiger organischer Rest ist, der durch Entfernung der primären Aminogruppen eines n-wertigen Amins erhalten wird,
R₁, R₂ gleiche oder verschiedene organische Reste sind, die keinen Zerewitinoffaktiven Wasserstoff aufweisen und
n eine ganze Zahl von mindestens 2 ist.
und
B) isocyanatfunktionelle Prepolymere mit Restmonomergehalten von weniger als 1 Gew.-% erhältlich durch Umsetzung von
B1)aliphatischen Isocyanaten mit
B2)einer Polyolkomponente mit zahlenmittleren Molekulargewichten von ≥ 400 g/mol und mittleren OH-Funktionalitäten von 2 bis 6, die Polyesterpolyole und/oder Polyester-Polyether-Polyole sowie gegebenenfalls Polyetherpolyole enthält.
oder
C) gegebenenfalls organische Füllstoffe, die eine nach DIN 53019 gemessenen Viskosität bei 23°C im Bereich von 10 bis 6000 mPas aufweisen und
D) gegebenenfalls Umsetzungsprodukte von isocyanatfunktionellen Prepolymeren gemäß der Definition der Komponente B) mit Asparaginsäureestern gemäß Komponente A) und/oder organischen Füllstoffen gemäß C).

2. Verwendung gemäß Anspruch 1, **dadurch gekennzeichnet, dass** die in B1) eingesetzten Polyetheresterpolyole eine Hydroxylzahl von 30 bis 140 mg KOH/g, eine Säurezahl von 0,05 bis 10 mg KOH/g und eine nach DIN 53019 gemessene Scherviskosität bei 23°C von 200 bis 8000 mPas aufweisen.

3. Verwendung gemäß Anspruch 1 oder 2, **dadurch gekennzeichnet, dass** in B2 Polyetherpolyole mit einem zahlenmittleren Molekulargewicht von 100 bis 2000 g/mol eingesetzt werden, wobei die Ethergruppen ganz oder teilweise von Ethylenoxid abgeleitet sind.

4. Verwendung gemäß Anspruch 3, **dadurch gekennzeichnet, dass** der Anteil an Polyetherpolyolen höchstens 50 Gew.-% bezogen auf die gesamte Komponente B2 beträgt.

5. Verwendung gemäß einem der Ansprüche 1 bis 4, **dadurch gekennzeichnet, dass** die Komponente B2 eine Estergruppenkonzentration (in Mol pro kg) von 0,5 bis 5,5 aufweist.

6. Verwendung gemäß einem der Ansprüche 1 bis 5, **dadurch gekennzeichnet, dass** es sich bei den organischen Füllstoffen der Komponente C) um hydroxyfunktionelle Verbindungen, bevorzugt Polyethylenglykole, handelt.

7. Verwendung nach einem der vorstehenden Ansprüche, **dadurch gekennzeichnet, dass** die Herstellung des Polyharnstoff-Systems durch Vermischen der Komponenten A), B), gegebenenfalls C) und gegebenenfalls D) erfolgt, wobei das Verhältnis von freien oder blockierten Aminogruppen zu freien NCO-Gruppen 1 : 1, das Gewichtsverhältnis der Füllstoffkomponente C) zur Aspartatkomponente A) 0 : 1 bis 20 : 1 beträgt.

8. Verwendung nach einem der vorstehenden Ansprüche, **dadurch gekennzeichnet, dass** das Polyharnstoff-System in einem 2-Kammerdosiersystem vorliegt, wobei eine Kammer das Prepolymer der Komponente A) und die andere die Härterkomponente B) und gegebenenfalls C) und D) umfasst.

9. Postoperative Adhäsionsbarriere erhältlich nach einer Verwendung gemäß einem der Ansprüche 1 bis 8.

## Claims

1. Use of a polyurea system for the production of a means for the production of a postoperative adhesion barrier, the polyurea system comprising
A) amino-functional aspartic esters of the general formula (I) 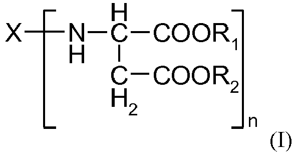 where
X is an n-valent organic radical obtained by removal of the primary amino groups of an n-functional amine,
R₁, R₂ are the same or different organic radicals devoid of any Zerevitinov-active hydrogen and
n is an integer of at least 2
and
B) isocyanate-functional prepolymers having residual monomer contents of less than 1% by weight obtainable by reaction of
B1) aliphatic isocyanates with
B2) a polyol component having number average molecular weights of ≥ 400 g/mol and average OH functionalities of 2 to 6 which contains polyester polyols and/or polyester-polyether polyols and also optionally polyether polyols,
or
C) optionally organic fillers having a DIN 53019 viscosity at 23°C in the range from 10 to 6000 mPas, and
D) where appropriate, reaction products of isocyanate-functional prepolymers as per the definition of component B) with aspartic esters as per component A) and/or organic fillers as per C).

2. Use according to Claim 1, **characterized in that** the polyether ester polyols used in B1) have a hydroxyl number of 30 to 140 mg KOH/g, an acid number of 0.05 to 10 mg KOH/g and a DIN 53019 shear viscosity at 23°C of 200 to 8000 mPas.

3. Use according to Claim 1 or 2, **characterized in that** B2 utilizes polyether polyols having a number average molecular weight of 100 to 2000 g/mol and having all or some of the ether groups derived from ethylene oxide.

4. Use according to Claim 3, **characterized in that** the proportion of polyether polyols comprises not more than 50% by weight based on the entire component B2.

5. Use according to any one of Claims 1 to 4, **characterized in that** the component B2 has an ester group concentration (in moles per kg) of 0.5 to 5.5.

6. Use according to any one of Claims 1 to 5, **characterized in that** the organic fillers of component C) comprise hydroxy-functional compounds, preferably polyethylene glycols.

7. Use according to any of the preceding Claims, **characterized in that** the preparation of the polyurea system is carried out by mixing the components A), B), where appropriate C) and where appropriate D), wherein the ratio of free or blocked amino groups to free NCO groups amounts to 1:1 the weight ratio of filler component C) to aspartate component A) ranges from 0:1 to 20:1.

8. Use according to any of the preceding Claims, **characterized in that** the polyurea system is present in a 2-chamber dispensing system, wherein one chamber comprises the prepolymer of component A) and the other chamber comprises the curative component B) and where appropriate C) and D)

9. Postoperative adhesion barrier obtainable by a use according to any one of Claims 1 to 8.

## Revendications

1. Utilisation d'un système de polyurée pour la fabrication d'un moyen de production d'une barrière d'adhésion postopératoire, le système de polyurée comprenant
A) des esters d'acide aspartique aminofonctionnels de formule générale (I) 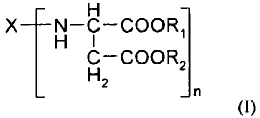 où
X est un groupe fonctionnel organique de valeur n qui est obtenu par extraction des groupes amino primaires d'une amine de valeur n,
R₁ et R₂ sont des groupes fonctionnels organiques identiques ou différents ne présentant aucun atome d'hydrogène actif de Zerewitinoff, et
n est un nombre entier équivalant à au moins 2,
et
B) des prépolymères fonctionnels isocyanate présentant une teneur en monomère résiduel de moins de 1 % en poids pouvant être obtenus par transformation
B1) d'isocyanates aliphatiques avec
B2) un composant polyol présentant des poids moléculaires moyens en nombre supérieurs ou égaux à 400 g/mol et des fonctionnalités OH moyennes de 2 à 6, qui contient des polyesterpolyols et/ou polyester-polyétherpolyols ainsi que, le cas échéant, des polyétherpolyols,
ou
C) le cas échéant, des matières de charge organiques présentant une viscosité mesurée à 23 °C selon DIN 53019 dans la gamme allant de 10 à 6000 mPas, et
D) le cas échéant, des produits de transformation de prépolymères fonctionnels isocyanate selon la définition du composant B) avec des esters d'acide aspartique selon le composant A) et/ou des matières de charge organiques selon C).

2. Utilisation selon la revendication 1, **caractérisée en ce que** les polyétheresterpolyols intervenant dans B1) présentent un indice d'hydroxyle de 30 à 140 mg KOH/g, un indice d'acide de 0,05 à 10 mg KOH/g et une viscosité de cisaillement mesurée à 23 °C selon DIN 53019 de 200 à 8000 mPas.

3. Utilisation selon la revendication 1 ou 2, **caractérisée en ce que**, dans B2, des polyétherpolyols présentant un poids moléculaire moyen en nombre de 100 à 2000 g/mol interviennent, les groupes éther étant dérivés intégralement ou partiellement de l'oxyde d'éthylène.

4. Utilisation selon la revendication 3, **caractérisée en ce que** la teneur en polyétherpolyols est au maximum 50 % en poids par rapport à la totalité du composant B2.

5. Utilisation selon l'une des revendications 1 à 4, **caractérisée en ce que** le composant B2 présente une concentration (en moles par kg) de groupes ester de 0,5 à 5,5.

6. Utilisation selon l'une des revendications 1 à 5, **caractérisée en ce que** les matières de charge organiques du composant C) sont des composés hydroxyfonctionnels, de préférence des polyéthylèneglycols.

7. Utilisation selon l'une des revendications précédentes, **caractérisée en ce que** la fabrication du système de polyurée s'effectue en mélangeant les composants A), B), le cas échéant C) et le cas échéant D), le rapport entre groupes amino libres ou bloqués et groupes NCO libres étant 1 : 1, le rapport de poids entre le composant de matières de charge C) et le composant aspartate A) étant 0 : 1 à 20 : 1.

8. Utilisation selon l'une des revendications précédentes, **caractérisée en ce que** le système de polyurée se trouve dans un système de dosage à deux compartiments, un compartiment comprenant le prépolymère du composant A) et l'autre le composant durcisseur B) et, le cas échéant, C) et D).

9. Barrière d'adhésion postopératoire pouvant être obtenue suivant une utilisation selon l'une des revendications 1 à 8.
